# EUROPÄISCHE PATENTSCHRIFT

(11) **EP 1 259 590 B1**
(45) Veröffentlichungstag und Bekanntmachung des Hinweises auf die Patenterteilung: **19.11.2008**
(21) Anmeldenummer: 01917042.2
(22) Anmeldetag: 28.02.2001
(51) Int. Cl.: C12N 5/00, C12N 15/87

(54) **VERFAHREN ZUM ÜBERTRAGEN VON MATERIAL IN EINEM ZELLSYSTEM**
METHOD FOR TRANSFERRING MATERIAL IN A CELL SYSTEM
PROCEDE POUR TRANSFERER DE LA MATIERE DANS UN SYSTEME CELLULAIRE

(30) Priorität: 06.03.2000 DE 10010959
(43) Veröffentlichungstag der Anmeldung: 27.11.2002
(73) Patentinhaber: EPPENDORF AG, 22339 Hamburg (DE)
(72) Erfinder: ZIMMERMANN, Ulrich, 97295 Waldbrunn (DE)
(74) Vertreter: Hertz, Oliver
(86) Internationale Anmeldenummer: PCT/EP2001/002252
(87) Internationale Veröffentlichungsnummer: WO 2001/066694

(56) Entgegenhaltungen:
- WO-A-00/09732
- WO-A-00/15032
- WO-A-00/22147
- A. EROGLU ET AL.: "Intracellular trehalose improves the survival of cryopreserved mammalian cells." NATURE BIOTECHNOLOGY, Bd. 18, Februar 2000 (2000-02), Seiten 163-167, XP002178806 NEW YORK, US

## Beschreibung

Die vorliegende Erfindung betrifft die Verwendung von Trehalose in einen Verfahren zum Übertragen von Material durch die Membran mindestens einer Zelle sowie die Anwendung dieses Verfahrens in der Gentechnik, Biotechnologie und Hybridomatechnik.

In den letzten Jahren haben Verfahren zum Übertragen von biologischen Materialien durch die Membran einer Zelle zunehmend an Bedeutung erlangt. Bei diesen Verfahren werden membranimpermeable Moleküle durch Poren, die sich durch äußere Kräfte in der Membran gebildet haben, geschleust. Diese Methoden haben den entscheidenden Vorteil, dass keine Vehikel verwendet werden müssen.

Die reversible Membranpermeabilisierung durch ein elektrisches Feld oder auch die Elektroporation ist seit einiger Zeit eine etablierte Methode für die Aufnahme freier DNA in beispielsweise Eukaryonten. Dabei setzt man die Eukaryonten in Gegenwart von DNA einem elektrischen Feld hoher Stärke aus. Man weiß allerdings nur sehr wenig über den Mechanismus der DNA-Aufnahme während der Elektroporation. Es wird angenommen, daß sich in der Zellmembran aufgrund des Elektroschocks vorübergehend Poren bilden und die DNA nach Kontakt mit der Lipiddoppelschicht der Zellmembran in die Zelle aufgenommen wird.

Anders als bei der Elektroporation, wo von außen Material in die Zelle eingeführt wird, ist die Elektrofusion zu betrachten, bei der mindestens zwei Zellen verschmelzen. Die Elektrofusion erfolgt mittels elektrischer Impulse in zwei Stufen. Zunächst werden die zu fusionierenden Zellen einem elektrischen Wechselfeld ausgesetzt, in dem sie sich infolge von Dielektrophorese gegenseitig anziehen. Die Leitfähigkeit des Mediums sollte möglichst gering sein. Im zweiten Schritt wird die Elektrofusion durch sehr kurze elektrische Gleichstrompulse ausgelöst. Dabei kommt es zu Interaktionen von Membranteilen, die zur Fusion führen. Mit dieser Methode können beispielsweise Protoplasten fusioniert werden. Es können auch Hybride tierischer Zellen, wie Hybridomazellen, und Hefen hergestellt werden.

So ist auch bereits seit einiger Zeit bekannt, eine Material-übertragung in eine Zelle dadurch zu bewirken, dass die Zellen zur Permeabilisierung mit Bestrahlung behandelt werden. Dabei werden die Zellen beispielsweise Laserstrahlung ausgesetzt, wonach dann das Material durch die Zellmembran diffundieren kann.

So sind auch bereits Methoden angewandt worden, bei denen die Membranen von Zellen für die Permeabilisierung mit chemischen Substanzen behandelt werden. Dazu gehören porenbildende und/oder diffusionsfördernde Peptid- und Depsipeptidantibiotika, wie Valinomycin, und Detergenzien, wie Natriumdodecylsulfat.

Die oben beschriebenen bekannten Verfahren basieren auf dem folgenden Grundprinzip: Durch energiereiche, elektrische, elektromagnetische und mechanische Kräfte, wie Stromimpulse, Bestrahlung, Ultraschall und Druck, und chemische Behandlung kommt es zu lokalen Öffnungen in der Zellmembran. Es bilden sich submikroskopische Löcher bzw. Poren. Eine derartige Behandlung ermöglicht das Einschleusen des biologischen Materials von außen oder, wenn zwei Zellen verschmolzen werden sollen, zwischen den Zellen. Nach Abschwächen der Intensität dieser von außen wirkenden Kräfte schließen sich die Poren in der Membran und das Material verbleibt in der Zelle.

Es hat sich allerdings bei diesen Verfahren herausgestellt, daß der Anteil an überlebenden Zellen, d.h. intakten, reversibel permeabilisierten Zellen, oftmals durch den Anteil toter Zellen übertroffen wird. Dieses ist darin zu sehen, dass die permeabilisierten Zellen nicht in der Lage sind, die Poren nach Abschwächung der äußeren Kräfte vollständig wieder zu schließen. Dabei kommt es dann zum Verlust wichtiger Zellfunktionen, so dass die Zelle nicht mehr in der Lage ist, ihren Stoffwechsel aufrecht zu erhalten, was letztlich zum Zelltod führt. Daher war es bei den Verfahren des Standes der Technik durchaus die Regel, mit einem Anteil toter Zellen rechnen zu müssen, was die Effizienz dieser Verfahren deutlich beeinträchtigte.

Es ist daher Aufgabe der vorliegenden Erfindung, ein Verfahren zum Übertragen von Material durch die Membran von Zellen zur Verfügung zu stellen, bei dem ein hoher Grad an reversibel permeabilisierten, sogenannten überlebenden Zellen erreicht wird und damit der Anteil an toten Zellen drastisch herabgesetzt wird. Die Erhöhung des Anteils an überlebenden Zellen soll auch dann erfolgen, wenn mit strikteren Reaktionsbedingungen, beispielsweise höheren Feldstärken, gearbeitet wird.

Diese Aufgabe wird mit der Verwendung von Trehalose gemäß Patentanspruch 1 oder 14 gelöst. Die Unteransprüche betreffen bevorzugte Ausführungs-formen der erfindungsgemäßen Verwendung.

Desweiteren werden in den Patentansprüchen besondere Anwendun-gen der erfindungsgemäßen Verwendung beschrieben.

Die vorliegende Erfindung betrifft ein Verfahren zum Übertragen von Material durch die Membran mindestens einer Zelle in einem wäßrigen Medium, welches dadurch gekennzeichnet ist, dass die Übertragung in Gegenwart von Trehalose durchgeführt wird.

Das Verfahren unter erfindungsgemäßer Verwendung von Trehalose läßt sich durch die Figuren näher erläutern. Es zeigen:
- Fig. 1: eine graphische Darstellung, worin die Propidiumiodid-Aufnahme und die Überlebensrate (% reversibel permeabilisierte Zellen) in Abhängigkeit der Feld-stärke im hypoosmolaren Medium gezeigt ist;
- Fig. 2: eine graphische Darstellung, worin die "pulse efficiency" als Maß für die Ausbeute in Abhängigkeit der Feldstärke im hypoosmolaren Medium angegeben ist;
- Fig. 3: eine graphische Darstellung, worin die Propidiumio-did-Aufnahme und die Überlebensrate (% reversibel permeabilisierte Zellen) in Abhängigkeit der Feld-stärke im isoosmolaren Medium gezeigt ist; und
- Fig. 4: eine graphische Darstellung, worin die "pulse efficiency" als Maß für die Ausbeute in Abhängigkeit der Feldstärke im isoosmolaren Medium angegeben ist;

Es hat sich erfindungsgemäß herausgestellt, dass die Überlebensrate von reversibel permeabilisierten Zellen drastisch erhöht wird, wenn dem wäßrigen Arbeitsmedium Trehalose zugeführt wird. Es wird angenommen, dass die Trehalose eine membranstabilisierende und membranheilende Funktion ausübt, wenn sich die Poren nach erfolgter Einschleusung des biologischen Materials wieder schließen. Das hat zur Folge, dass die permeabilisierten Zellen überleben und somit nicht die Gefahr gegeben ist, dass durch den Verlust von Zellflüssigkeiten und -organellen die Zellfunktionen absterben.

Die Verwendung von Trehalose in Verfahren der hier beschriebenen Art ist bisher noch nicht bekannt gewesen. Die Literatur gibt lediglich Hinweise darauf, dass die Trehalose bei der Cryokonservierung von Säugetierzellen (Nature Biotechnology, Vol. 18, Februar 2000) und bei der Aufrechterhaltung intakter menschlicher Zellen ohne die Anwesenheit von Wasser (Nature Biotechnology, Vol 18, Februar 2000) einen Beitrag leistet.

Auch in der internationalen Patentanmeldung WO 00/09732 wird lediglich die Verwendung von Trehalose als Gefrierschutzmittel offenbart, nicht jedoch die Verwendung als membranheilendes Mittel in einem wässrigen Medium ohne Gefriervorgänge.

Als Trehalose, die in den jeweiligen Arbeitspuffern gelöst wird, kann prinzipiell jede Trehalose verwendet werden. Die Trehalose ist ein Disaccharid und kommt in der Natur vor und wird auch in einem Fall synthetisch hergestellt. Die bekannteste und am häufigsten in der Natur vorkommende Trehalose ist die α,α-Trehalose. Ebenfalls in der Natur vorkommende Trehalose ist die α,β-Trehalose, die man in Honig nachgewiesen hat. Die β,β-Trehalose ist nur synthetisch zugänglich. Vorzugsweise setzt man die α,α-Trehalose dem Arbeitspuffer bei der Übertragung des Materials zu.

Das Verfahren unter erfindungsgemäßer Verwendung von Trehalose eignet sich vorzugsweise für die Übertragung von Material unter Beteiligung mindestens einer Zelle, die reversibel permeabilisiert wird oder unter Beteiligung von mindestens zwei aneinander haftender Zellen, die permeabilisiert werden und praktisch Material untereinander austauschen. Hier ist es möglich, dass mindestens zwei Zellen miteinander verschmelzen, wobei es auch die Varianten gibt, daß mehrere Zellen, quasi unter Bildung eines Perlenstrangs, miteinander fusionieren.

Üblicherweise erfolgt die Übertragung von Material in die Zelle durch lokale Öffnung bzw. Öffnungen der Membran der Zelle bzw. der Zellen. In diesem Fall spricht man von einer Permeabilisierung der Membran. Es ist davon auszugehen, dass durch einen Teil der Öffnungen das Material übertragen wird und der andere Teil der Öffnungen ohne Materialdurchgang verbleibt. Auch diese Öffnungen müssen sich nach Aufnahme des Materials wieder schließen, wobei sich gerade hier insbesondere die Tre-halose als membranheilendes Additiv als besonders vorteilhaft erwiesen hat.

Die Permeabilisierung der Membran kann in der Regel durch Anlegen eines elektrischen Felds, durch Bestrahlung oder chemische Behandlung erfolgen. Welche Methode hier gewählt wird, hängt im wesentlichen davon ab, welche Zellen transformiert werden sollen und welches Material übertragen werden soll.

Für die elektrische Permeabilisierung eignen sich Methoden, zu denen die Elektrotransfektion, Elektroporation und Elektrofusion sowohl in makroskopischen Vorrichtungen als auch in Mikrosystemen/Mikrostrukturen gehören. Hier handelt es sich um etablierte Verfahren, mit denen bereits seit einigen Jahren erfolgreich in der Gentechnik gearbeitet wurde. Insbesondere bei der Elektroporation ist es oftmals erforderlich, bei hohen Feldstärken zu arbeiten. Herkömmlicherweise ergab sich hier aber das Problem, dass die Zellen dann abstarben, d.h. irreversibel permeabilisiert worden zu sein. Dieses hat dann zu stark verminderten Ausbeuten geführt. Wenn man aber erfindungsgemäß dem Elektroporationspuffer Trehalose zufügt, ist es möglich, auch bei hohen Feldstärken reversibel permeabilisierte lebende Zellen zu erhalten. Dieses macht das Verfahren weitaus wirtschaftlicher.

So ist auch eine Permeabilisierung durch UV-oder Laserbestrahlung möglich. Bestrahlungen dieser Art sind dann vorteilhaft, wenn sich aufgrund der verwendeten Zellen und des zu übertragenden Materials eine elektrische Behandlung nicht anbietet.

So ist eine chemische Behandlung zur Permeabilisierung nur dann empfehlenswert, wenn es nicht ratsam ist, eine Übertragung im elektrischen Feld oder durch Bestrahlung durchzuführen. Sollte eine chemische Behandlung gewählt werden, so sind die Zellen für die Permeabilisierung mit Antibiotika, Detergenzien, etc. zu behandeln.

Mit dem Verfahren unter erfindungsgemäßer Verwendung von Trehalose kann als Material jedes geeignete biologische Material übertragen werden. Dazu zählen: Xenomoleküle, DNA und RNA, Plasmide, Chromosomen, Chromosomenteile und künstliche Chromosomen, Proteine und Glykoproteine, Zellen, Zellteile und Zellorganellen oder niedrigmolekulare Fremdstoffe.

Das biologische Material kann Trehalose bzw. Trehalose im Gemisch mit Saccharose selbst sein. Auf diese Weise ist es möglich, Trehalose bzw. das Trehalose/Saccharose-Gemisch als intrazelluläres Cryoprotektans oder Schutz vor Austrocknung einzuschleusen.

Das vorliegende Verfahren ist hinsichtlich der bei der Übertragung verwendeten Zellen keinen Beschränkungen unterlegen. Es ist möglich, für die Übertragung von Material natürliche Zellen oder membranumhüllte Vesikel zu verwenden. Als Beispiele für membranumhüllte Vesikel können natürliche oder künstliche Vesikel, Liposomen und Micellen genannt werden.

Als natürliche Zellen lassen sich erfindungsgemäß prokaryontische und eukaryontische Zellen permeabilisieren und transformieren. Als prokaryontische Zellen sind Bakterien, Blaualgen und Archaebakterien zu nennen. Die eukaryontischen Zellen können ihren Ursprung in Protozoen, Pflanzen (einschließlich Algen), Pilzen (einschließlich Hefen), Tieren oder Menschen haben.

Bei der Elektroporation und Elektrofusion kann im isoosmolaren Medium und auch im hypoosmolaren Medium gearbeitet werden. Das hypoosmolare Medium weist bei tierischen Zellen eine Osmolarität von 75 bis 250 mOsm auf und ist daher unphysiologisch. Das isoosmolare Medium weist eine Osmolarität von etwa 300 mOsm auf und entspricht physiologischer Umgebung. Bei Pflanzenzellen hingegen spricht man bei etwa 500 mOsm von einem isoosmolaren Medium. Das hypoosmolare Medium bewegt sich bei etwa 400 bis 450 mOsm.

Es hat sich herausgestellt, daß gerade im hypoosmolaren Medium der Schutzeffekt der Trehalose deutlich wird.

Es hat sich gezeigt, daß die Konzentration der Trehalose im wäßrigen Medium bei beispielsweise elektrischer Behandlung im Bereich von 1 bis 200 mM liegen soll. Es ist festgestellt worden, dass Trehalose den Anteil überlebender Zellen nach Pulsapplikation erhöht, wobei bei ca. 30 bis 50 mM ein Optimum in der Ausbeute auftritt, der sich bei weiterer Konzentrationserhöhung nicht verstärkt. Daher ist bei der Pulsapplikation ein Konzentrationsbereich von etwa 30 bis 50 mM bevorzugt.

Es hat sich herausgestellt, daß das vorliegende Verfahren in einer weiteren Ausführungsform auch dann zu einer erhöhten Ausbeute an reversibel permeabilisierten Zellen führt, wenn dem Arbeitspuffer ein Gemisch aus Trehalose und Saccharose zugesetzt wird. Das Verhältnis von Trehalose zu Saccharose liegt im Bereich 1:2 bis 1:10. Die Konzentration des Gemischs liegt im Bereich von 200 bis 300 mM.

Das vorliegende Verfahren ist hervorragend zum Einschleusen von biologischem Material in eine Zelle oder zwischen mindestens zwei Zellen geeignet. Daher ist es praktisch auf allen Gebieten der Biotechnologie, Gentechnik und Mikrosystemtechnik einsetzbar. Inbesondere läßt sich eine besondere Eignung in der Hybridomatechnik unter Anwendung beispielsweise der Elektrofusion erkennen. Auch lassen sich ohne weiteres mit dem Verfahren pflanzliche Protoplasten fusionieren.

Das vorliegende Verfahren hat durch die Gegenwart von Trehalose im Arbeitsmedium eine Vielzahl von Vorteilen. Diese sind darauf zurückzuführen, dass die Trehalose auf die zu behandelnden Zellen einen Schutzeffekt ausübt. Die Trehalose stabilisiert die Zellmembran und bewirkt beispielsweise nach Aufnahme des Fremdmaterials und Abschwächung der äußeren Kräfte die schnelle Ausheilung der Poren. Insbesondere hat sich bei der Elektroporation herausgestellt, dass der Schutzeffekt der Trehalose bei hohen Feldstärken stark ausgeprägt ist, während er von der Pulsdauer kaum beeinflußt ist. Es ist festgestellt worden, dass die schützende Wirkung der Trehalose im hypotonen Pulsmedium stärker auftritt als im isotonen Pulsmedium. Im schwach leitenden Medium ist die Wirkung der Trehalose etwas stärker ausgebildet als im höher leitenden Medium. Diese vorteilhaften Schutzeigenschaften der Trehalose sind insbesondere dann von größtem Nutzen, wenn bei strikten Pulsbedingungen, wie geringer Leitfähigkeit, hypotonem Stress, hoher Feldstärke, gearbeitet wird.

Nachfolgend wird das Verfahren unter erfindungsgemäßer Verwendung von Trehalose anhand der Beispiele näher erläutert.

### Beispiele

### Beispiel 1:

Elektroporation von Zellen mit und ohne Trehalose im hypoosmolaren Arbeitsmedium.

Als Pulsmedium wurde ein Phosphatpuffer mit 1,15 mM K₂HPO₄/KH₂PO₄-Puffer, pH 7,2 verwendet. Als Leitsalz wurde KCl in einer Konzentration von 10 mM (σ = 1,5 - 1,6 mS/cm) hinzugesetzt. Dann wurde Trehalose in entsprechenden Konzentrationen dem Pulsmedium hinzugefügt. Die Osmolarität wurde durch Zugabe von Inosit auf 100 mOsm eingestellt, um eine hypoosmolare Lösung zu erhalten.

Als Zellen wurden Jurkat-Zellen verwendet, welche Zellen einer humanen T-Lymphozyten-Linie sind.

Als zu übertragendes Material wurden 40 µg/ml Propidiumiodid, welches ein membranimpermeabler DNA-Farbstoff ist, hinzugesetzt.

Die Pulsapplikation erfolgte nach zehnminütiger Inkubation vor Pulsapplikation im Pulsmedium bei Raumtemperatur (Zelldichte: 2-3 x 10⁶ Zellen/ml). Die Pulsdauer betrug 20 µs.

Die Elektroporation erfolgte in einem Eppendorf-Multiporator. Nach Pulsgabe ließ man 10 min bei Raumtemperatur die Poren schließen (Resealing).

Die Elektropermeabilisierung erfolgte bei folgenden Trehalosekonzentrationen: 0 mM und 40 mM (α,α-Trehalose).

Es wird bei 4°C oder Raumtemperatur gepulst. Es kann einfach gepulst werden, während sich aber ein mehrfaches Pulsen, bis zu 3, als vorteilhaft erweisen kann.

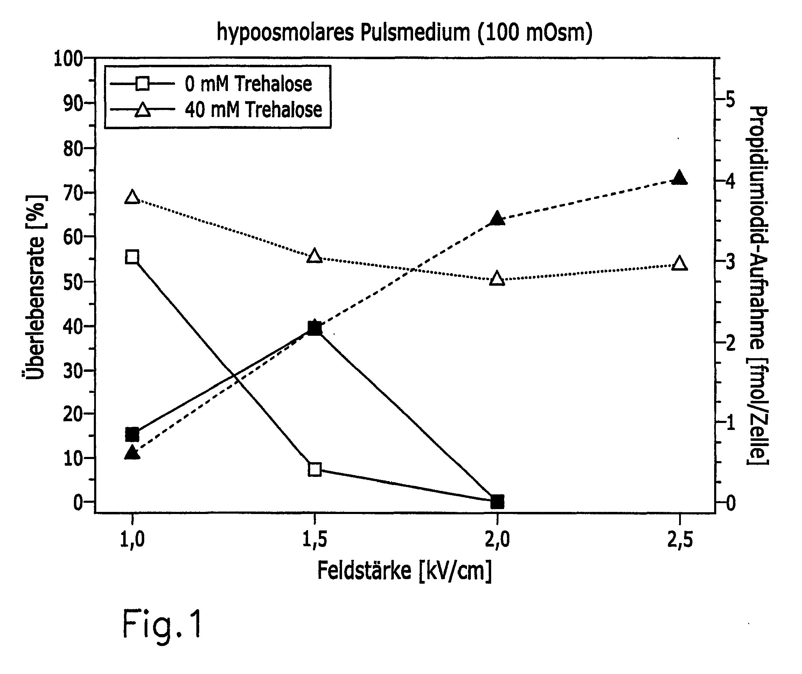

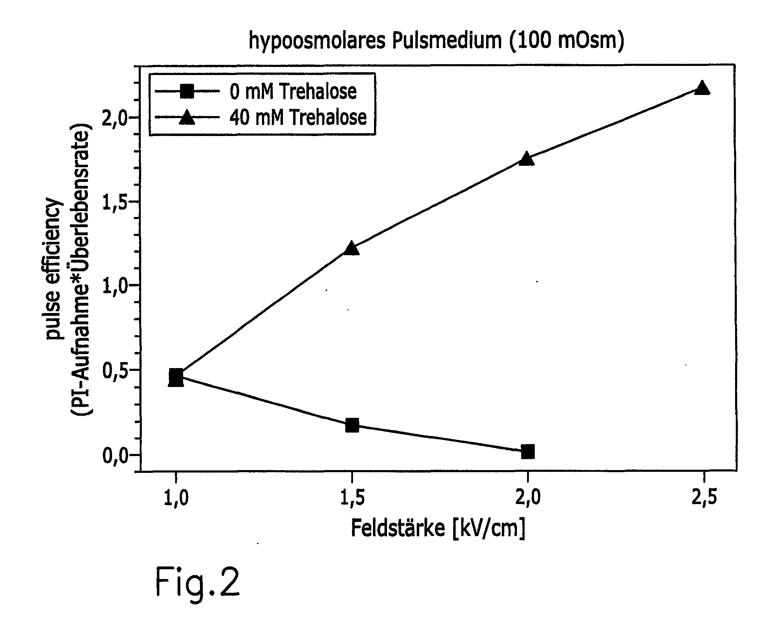

Die Ergebnisse sind in den Figuren 1 und 2 dargestellt.

Figur 1 zeigt graphisch die Aufnahme von Propidiumiodid bzw. die Überlebensrate der elektropermeabilisierten Zellen in Abhängigkeit der Feldstärke. Die Poration ohne Trehalose ist mit Quadraten dargestellt. Der Ansatz mit 40 mM Trehalose wird in der Darstellung mit Dreiecken angeben. Die offenen Symbole zeigen die Überlebensrate (% Anteil der reversibel permeabilisierten Zellen), während die gefüllten Symbole die Propidiumiodid-Aufnahme in die Zelle darstellen.

Wie aus Figur 1 zu entnehmen ist, erleiden die Zellen in einem Pulsmedium ohne Trehalose bei Feldstärken ab 1,5 kV/cm irreversibele Schädigungen und sterben durch Verlust der Zellfunktionen ab (offene Quadrate), wogegen in einem Pulsmedium im 40 mM Trehalose selbst bei sehr hohen Feldstärken bis 2,5 kV/cm nur ein relativ geringer Anteil von toten Zellen zu verzeichnen sind (offene Dreicke). Dies ist ein Hinweis darauf, dass die Zellen reversibel permeabilisiert wurden und lebensfähig geblieben sind. Die Propidiumiodid-Aufnahme wird nur in geringem Maße durch Trehalose beeinflusst.

In Figur 2 wird die "pulse efficiency", welche das Produkt von Propidiumiodid-Aufnahme und Überlebensrate darstellt, als Maß für die Ausbeute der Eletroporation in Abhängigkeit der Feldstärke untersucht. Hier ist festzustellen, dass diese Werte in Gegenwart von 40 mM Trehalose im Pulsmedium (Dreiecke) mit Erhöhung der Feldstärke stark ansteigen und deutlich über den Werten einer nicht mit Trehalose behandelten Kontrolle (Quadrate) liegen. Diese deutliche Erhöhung der Ausbeute lässt sich ebenfalls auf eine stark verbesserte Überlebensrate in Gegenwart von 40 mM Trehalose zurückführen.

### Beispiel 2:

Elektroporation von Zellen mit und ohne Trehalose im isoosmolaren Arbeitsmedium.

Es werden im wesentlichen die gleichen Versuchsbedingungen wie im Beispiel 1 angewendet, mit der Ausnahme, dass die Osmolarität des Pulsmediums durch Zugabe von Inosit auf 290 mOsm auf isoosmolare Bedingungen eingestellt worden ist.

Es wurde wieder in das Pulsmedium α,α-Trehalose in folgenden Konzentrationen eingegeben: 0 mM und 40 mM.

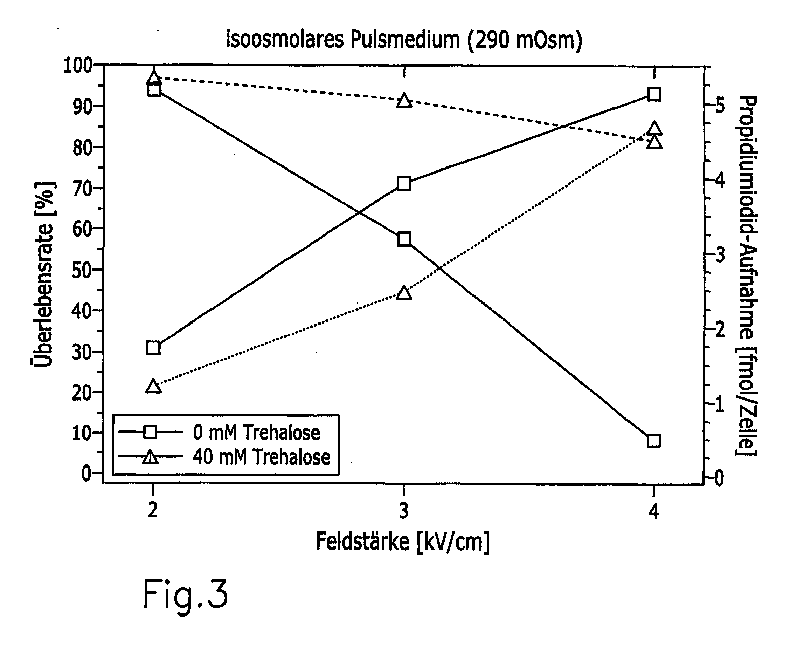

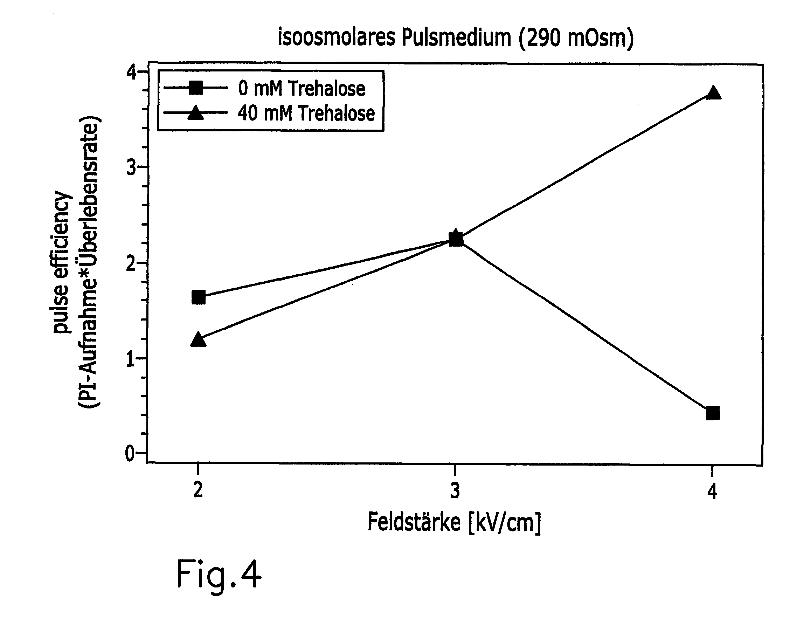

Die Ergebnisse sind aus den Figuren 3 und 4 zu entnehmen.

Fig. 3 zeigt die Abhängigkeit der Überlebensrate und der Propidiumiodid-Aufnahme gegenüber der Feldstärke. Die offenen Symbole stehen für die Überlebensrate, die gefüllten für die Propidiumiodid-Aufnahme.

Im isomolaren Pulsmedium ist die Überlebensrate bei den unbehandelten Zellen (0 mM, offene Quadrate) um ein Vielfaches geringer als bei mit 40mM Trehalose behandelten Zellen (offene Dreiecke), vor allem bei hohen Feldstärken. Die Propidiumiodid-Aufnahme variiert dagegen weniger stark. Es ist zu beachten, dass ein Absterben der Zellen ab einer Feldstärke von 3 kV/cm drastisch zu bemerken ist, wenn im Pulsmedium keine Trehalose vorhanden ist.

Die "pulse efficiency" wird in Figur 4 in Abhängigkeit der Feldstärke für die vorliegenden Pulsmedien (0 bis 40 mM Trehalose) gezeigt. Im Trehalose-haltigen Pulsmedium (Dreiecke) ist mit steigender Feldstärke eine stetige Erhöhung der "pulse efficiency" zu beobachten, während bei Fehlern der Trehalose (Quadrate) im Pulsmedium bei hohen Feldstärken eine drastische Reduzierung der Ausbeute zu erkennen ist. Auch hier zeigt die Trehalose wieder ihren Schutzeffekt bei strikten Pulsbedingungen.

## Patentansprüche

1. Verwendung von Trehalose zur Erhöhung der Überlebensrate von reversibel permeabilisierten Zellen während des Übertragens von Material durch die Membran mindestens einer Zelle in einem wässrigen Medium,
**dadurch gekennzeichnet, dass** die Übertragung in vitro durch Permeabilisierung der Zellmembran in Gegenwart von 1 bis 200 mM Trehalose durchgeführt wird.

2. Verwendung nach Anspruch 1, **dadurch gekennzeichnet, dass** die Konzentration der Trehalose 30 bis 50 mM beträgt.

3. Verwendung nach Anspruch 1 oder 2, **dadurch gekennzeichnet, dass** die Übertragung des Materials von außen in die Zelle erfolgt oder das Material zwischen mindestens zwei Zellen übertragen wird.

4. Verwendung nach mindestens einem der Ansprüche 1 bis 3, **dadurch gekennzeichnet, dass** die Membran durch Anlegen eines elektrischen Feldes, Bestrahlung oder chemische Behandlung permeabilisiert wird.

5. Verwendung nach Anspruch 4, **dadurch gekennzeichnet, dass** für die Permeabilisierung elektrische Methoden angewendet werden, zu denen die Elektrotransfektion, Elektroporation und Elektrofusion sowohl in makroskopischen Vorrichtungen als auch in Mikrosystemen/Mikrostrukturen gehören.

6. Verwendung nach Anspruch 4, **dadurch gekennzeichnet, dass** die Membran durch UV- oder Laserbestrahlung permeabilisiert wird.

7. Verwendung nach Anspruch 4, **dadurch gekennzeichnet, dass** die Membran durch Behandlung mit Antibiotika oder Detergenzien permeabilisiert wird.

8. Verwendung nach Anspruch 1, **dadurch gekennzeichnet, dass** als Material biologisches Material übertragen wird.

9. Verwendung nach Anspruch 8, **dadurch gekennzeichnet, dass** als biologisches Material Xenomoleküle, DNA und RNA, Plasmide, Chromosomen, Chromosomenteile und künstliche Chromosomen, Proteine und Glycoproteine, Zellen, Zellteile und Zellorganellen oder niedermolekulare Fremdstoffe, Trehalose oder ein Trehalose/Saccharose-Gemisch übertragen werden.

10. Verwendung nach mindestens einem der Ansprüche 1 bis 9, **dadurch gekennzeichnet, dass** die bei der Übertragung verwendeten Zellen natürliche Zellen oder membranumhüllte Vesikel sind.

11. Verwendung nach Anspruch 10, **dadurch gekennzeichnet, dass** als natürliche Zellen prokaryontische und eukaryontische Zellen verwendet werden.

12. Verwendung nach Anspruch 11, **dadurch gekennzeichnet, dass** die eukaryontischen Zellen menschlichen, tierischen oder pflanzlichen Ursprungs sind.

13. Verwendung nach Anspruch 1, **dadurch gekennzeichnet, dass** als wässriges Medium ein isoosmolares oder hypoosmolares Medium verwendet wird.

14. Verwendung von Trehalose zur Erhöhung der Überlebensrate von reversibel permeabilisierten Zellen während des Übertragens von Material durch die Membran mindestens einer Zelle in einem wässrigen Medium,
**dadurch gekennzeichnet, dass** die Übertragung in vitro durch Permeabilisierung der Zellmembran in Gegenwart eines Gemisches von Trehalose und Saccharose im Verhältnis 1:2 bis 1:10 (Trehalose:Sacharose) durchgeführt wird und die Trehalose in einer Konzentration von 200 bis 300 mM vorliegt.

15. Verwendung nach Anspruch 1 oder 14 zum Einschleusen von biologischem Material in eine Zelle.

16. Verwendung nach Anspruch 15, **dadurch gekennzeichnet, dass** das biologische Material Trehalose oder Trehalose im Gemisch mit Saccharose selbst ist.

17. Verwendung nach Anspruch 16 auf dem Gebiet der Gentechnik, Biotechnologie, Hybridomatechnik und Mikrosystemtechnik.

## Claims

1. Use of trehalose in order to increase the survival rate of reversibly permeabilised cells during the transfer of material through the membrane of at least one cell in an aqueous medium,
**characterised in that** the transfer is implemented in vitro by means of permeabilising the cell membrane in the presence of 1 to 200 mM trehalose.

2. Use according to claim 1, **characterised in that** the concentration of the trehalose is 30 to 50 mM.

3. Use according to claim 1 or 2, **characterised in that** the transfer of the material is effected from the exterior into the cell or the material is transferred between at least two cells.

4. Use according to at least one of the claims 1 to 3, **characterised in that** the membrane is permeabilised by application of an electrical field, irradiation or chemical treatment.

5. Use according to claim 4, **characterised in that,** for permeabilisation, electrical methods are applied, which include electrotransfection, electroporation and electrofusion both in macroscopic devices and in microsystems/microstructures.

6. Use according to claim 4, **characterised in that** the membrane is permeabilised by UV or laser irradiation.

7. Use according to claim 4, **characterised in that** the membrane is permeabilised by treatment with antibiotics or detergents.

8. Use according to claim 1, **characterised in that** biological material is transferred as material.

9. Use according to claim 8, **characterised in that** xenomolecules, DNA and RNA, plasmids, chromosomes, chromosome parts and artificial chromosomes, proteins and glycoproteins, cells, cell parts and cell organelles or low-molecular extraneous materials, trehalose or a trehalose/saccharose mixture are transferred as biological material.

10. Use according to at least one of the claims 1 to 9, **characterised in that** the cells used during the transfer are natural cells or vesicles which are surrounded by a membrane.

11. Use according to claim 10, **characterised in that** prokaryotic and eukaryotic cells are used as natural cells.

12. Use according to claim 11, **characterised in that** the eukaryotic cells are of human, animal or vegetable origin.

13. Use according to claim 1, **characterised in that** an isoosmolar or hypoosmolar medium is used as aqueous medium.

14. Use of trehalose in order to increase the survival rate of reversibly permeabilised cells during the transfer of material through the membrane of at least one cell in an aqueous medium,
**characterised in that** the transfer is implemented in vitro by means of permeabilising the cell membrane in the presence of a mixture of trehalose and saccharose in the ratio 1 : 2 to 1 : 10 (trehalose : saccharose), and the trehalose is present in a concentration of 200 to 300 mM.

15. Use according to claim 1 or 14, for the introduction of biological material into a cell.

16. Use according to claim 15, **characterised in that** the biological material is trehalose or trehalose in a mixture with saccharose itself.

17. Use according to claim 16 in the field of gene technology, biotechnology, hybridoma technology and microsystem technology.

## Revendications

1. Utilisation du tréhalose pour augmenter le taux de survie des cellules perméabilisées de manière réversible pendant le transfert de la matière à travers la membrane d'au moins une cellule dans un milieu aqueux,
**caractérisée en ce que** le transfert s'effectue *in vitro* par perméabilisation de la membrane cellulaire en présence de 1 à 200 mM de tréhalose.

2. Utilisation selon la revendication 1, **caractérisée en ce que** la concentration en tréhalose est de 30 à 50 mM.

3. Utilisation selon la revendication 1 ou 2, **caractérisée en ce que** le transfert de la matière s'effectue depuis l'extérieur dans la cellule ou **en ce que** la matière est transférée entre au moins deux cellules.

4. Utilisation selon au moins l'une des revendications 1 à 3, **caractérisée en ce que** la membrane est perméabilisée par application d'un champ électrique, par irradiation ou traitement chimique.

5. Utilisation selon la revendication 4, **caractérisée en ce que**, pour la perméabilisation, on utilise des méthodes électriques dont font partie l'électrotransfection, l'électroporation et l'électrofusion aussi bien dans des dispositifs macroscopiques que dans des microsystèmes/microstructures.

6. Utilisation selon la revendication 4, **caractérisée en ce que** la membrane est perméabilisée par irradiation d'UV ou laser.

7. Utilisation selon la revendication 4, **caractérisée en ce que** la membrane est perméabilisée par traitement avec des antibiotiques ou des détergents.

8. Utilisation selon la revendication 1, **caractérisée en ce que**, comme matière, on transfert de la matière biologique.

9. Utilisation selon la revendication 8, **caractérisée en ce que**, comme matière biologique, on transfère des xénomolécules, de l'ADN et de l'ARN, des plasmides, des chromosomes, des parties de chromosomes et des chromosomes artificiels, des protéines et des glycoprotéines, des cellules, des parties de cellules et des organelles cellulaires ou des substances étrangères de faible poids moléculaire, du tréhalose ou un mélange tréhalose/saccharose.

10. Utilisation selon au moins l'une des revendications 1 à 9, **caractérisée en ce que** les cellules utilisées pour le transfert sont des cellules naturelles ou des vésicules enveloppées d'une membrane.

11. Utilisation selon la revendication 10, **caractérisée en ce que** l'on utilise comme cellules naturelles des cellules procaryotes et eucaryotes.

12. Utilisation selon la revendication 11, **caractérisée en ce que** les cellules eucaryotes sont d'origine humaine, animale ou végétale.

13. Utilisation selon la revendication 1, **caractérisée en ce que** l'on utilise comme milieu aqueux un milieu iso-osmolaire ou hypo-osmolaire.

14. Utilisation du tréhalose pour augmenter le taux de survie des cellules perméabilisées de manière réversible pendant le transfert de la matière à travers la membrane d'au moins une cellule dans un milieu aqueux,
**caractérisée en ce que** le transfert s'effectue *in vitro* par perméabilisation de la membrane cellulaire en présence d'un mélange de tréhalose et de saccharose en un rapport de 1/2 à 1/10 (tréhalose/saccharose) et que le tréhalose est présent en une concentration de 200 à 300 mM.

15. Utilisation selon la revendication 1 ou 14 pour l'inclusion de matière biologique dans une cellule.

16. Utilisation selon la revendication 15, **caractérisée en ce que** la matière biologique est le tréhalose ou le tréhalose en mélange avec du saccharose lui-même.

17. Utilisation selon la revendication 16 dans le domaine de la génétique, de la biotechnologie, de la technique des hybridomes et de la technique des microsystèmes.
